# EUROPEAN PATENT SPECIFICATION

(11) **EP 4 243 688 B1**
(45) Date of publication and mention of the grant of the patent: **31.12.2025**
(21) Application number: 21807289.0
(22) Date of filing: 15.11.2021
(51) Int. Cl.: A61B 5/145, A61B 5/00, G16H 20/17, G16H 40/63, A61M 5/172, G16H 20/13, A61M 5/142

(54) **SMART BRACELET DEVICE CONTAINING GLUCOSE AND/OR GLUCAGON**
INTELLIGENTE ARMBANDVORRICHTUNG MIT GLUCOSE UND/ODER GLUCAGON
DISPOSITIF DE BRACELET INTELLIGENT CONTENANT DU GLUCOSE ET/OU DU GLUCAGON

(30) Priority: 16.11.2020 IT 202000027375
(43) Date of publication of application: 20.09.2023
(73) Proprietor: Cucchiarelli, Cristina, 00119 Roma (RM) (IT)
(72) Inventor: Cucchiarelli, Cristina, 00119 Roma (RM) (IT)
(74) Representative: Corradini, Corrado
(86) International application number: PCT/IB2021/060542
(87) International publication number: WO 2022/101865

(56) References cited:
- US-A1- 2010 004 522
- US-B2- 10 549 037
- P. RANDINE ET AL: "The House of Carbs: Personalized Carbohydrate Dispenser for People with Diabetes.", DOI: 10.3233/SHTI200249., vol. 270, 16 June 2020 (2020-06-16), PMID: 32570472, pages 693 - 697, XP002803826, Retrieved from the Internet <URL:https://pubmed.ncbi.nlm.nih.gov/32570472/> [retrieved on 20210712], DOI: 10.3233/SHTI200249.

## Description

### Field of the art

The present invention operates in the field of integrated devices and systems for protecting people. More in detail, within this industrial field, the finding described and claimed hereinbelow specifically regards a wearable device for monitoring and protection from hypoglycemia events.

### Prior art

At the state of the art, there are a variety of devices which allow monitoring the concentration of glucose in the blood.

It is in fact of fundamental importance, in subjects affected by pathologies such as diabetes, to constantly monitor the glucose levels thereof and supplement them in a quick/sudden manner if necessary, in order to prevent the occurrence of a situation of hypoglycemia.

According to data published in 2017 by the World Diabetes Federation, worldwide there are over 415 million people who live with diabetes and this number is destined to increase to 642 million in 2040. The consequences of only one episode of severe hypoglycemia can have considerable consequences on cerebral functionality and constant hypoglycemic crises are associated with an increased risk of cardiovascular events and mortality. According to an English study of 1979, hypoglycemia would be responsible for 4% of deaths of people under 50 with diabetes; a recent Norwegian study instead estimates on the order of 8% the predominance of deaths directly linked to hypoglycemia in a type 1 diabetic population under 56 years old. The studies conducted worldwide on subjects with type 1 and 2 diabetes have detected a risk of cardiovascular events and mortality increased by 1.5 to 6 times in subjects with hypoglycemic crises. This association is present both in subjects under therapy with insulin and in those under treatment with sulfonylurea.

Particularly at risk are elderly patients (> 75 years) with diabetes, and fragile subjects with comorbidity and chronic pathologies in terminal phase. In this category, the benefits of a very strict metabolic control have not been demonstrated; on the contrary, this could expose them to increased risk, especially tied to hypoglycemia. This information is very well-known, yet notwithstanding this it is quite common that these patients are hyper-treated and this increases the risk of hypoglycemia (already per se associated with age), of a series of morbidities, of a reduction of the cognitive performances and of renal functionality.

In addition, severe hypoglycemic crises are quite frequent in pregnant diabetic women treated with insulin, especially in the first trimester and in the under 18 group the most probable effect of hypoglycemia on the cardiovascular system is sudden death during sleep (dead-in-bed-syndrome).

The evidence that hypoglycemia can determine heart dysfunction and sudden death (e.g. from arrythmias) is increasingly convincing. Hypoglycemia activates the sympathetic nervous system and determines the release of catecholamines which in turn increase the heart rate, the contractility and the cardiac output; the plasmatic potassium is quickly reduced, determining electrophysiological alterations and ECG alterations. Other alterations can also occur: of the coagulation, endothelial dysfunction and release of inflammatory cytokines; all alterations which can lead to intravascular coagulation and thrombosis. The hemorheological and inflammatory alterations induced by hypoglycemia can last for days in subjects with type 2 diabetes and the functional alterations, which persist even after the restoration of a normal glycemia, can determine an intravascular environment favorable for a thrombosis event. In subjects with type 2 diabetes, therefore, these alterations associated with hypoglycemia could be responsible for acute cardiovascular events, such as ischemia and heart attack, heart failure, heart arrythmias.

Hypoglycemia also leads to neuronal damage. The regions hit by a hypoglycemic crises are the cortex, the neostriatum and the hippocampus which can reach neuronal death. Hypoglycemia is an important risk factor for cognitive deterioration. Slight moderate hypoglycemia (70-40) produces a neuron shock; severe hypoglycemia can determine neuronal death. The presence of diabetes worsens the neuronal damage and the cognitive deficit induced by hypoglycemia.

This condition can also have considerable psychological effects, both on the patient and on his/her family members. In addition to negatively affecting the quality of life, the patient (or his/her family members) live in fear that this could repeat itself. This is the case for parents of children with type 1 diabetes (for which the episodes of nocturnal hypoglycemia of their children represent violent emotional stress), as with subjects with reduced awareness of the hypoglycemia episodes, which can lead to convulsions and coma without premonitory symptoms or with subjects who have experienced a hypoglycemia episode in public and are very much embarrassed by the event. Several patients react by maintaining the glycemia levels higher, in order to avoid further hypoglycemic crises, but in such a manner exposing themselves to poor metabolic control and without informing the doctor of this poor compliance with the therapies.

For those affected with diabetes who wish to undertake a physical activity, hypoglycemia can represent a serious risk: when the level of glucose in the blood falls below 40-50 mg/dL, in fact, one can even reach so-called hypoglycemic coma, which is generally manifested after an excessive physical force, following dietetic errors (late consumption or non-consumption of food), after insulin or oral glycemia-lowering drug administration errors, following situations that prolong the glycemia-lowering action of the insulin or of oral antidiabetic substances (reduced renal functionality or association with other drugs). The symptoms that precede the hypoglycemic coma are: dizziness, fatigue, sensation of hunger, sweating, tachycardia, tremors, agitation, headaches, lip formication, speech difficulty. Hypoglycemia can also appear even several hours after exercise.

In order to limit the onset of hypoglycemia events, presently there are many different monitoring systems such as, for example, the device "CGM Dexcom G5 Mobile" which is a continuous glycemia monitoring device recommended for detecting glycemic trends and patterns in adults and in children and young adults of pediatric age (2-17 years old) with Type 1 and Type 2 diabetes subjected to insulin therapy.

The CGM is composed of an electrochemical sensor positioned below the skin of the abdomen which uninterruptedly measures up to 288 levels of glucose each day (one every 5 minutes). It is provided with support base for housing the Dexcom G5 Mobile transmitted and the suitable applicator which facilitates the insertion thereof and renders it painless. The sensor G5 Mobile is a sterile disposable device with 7 day duration

Another example is the patent WO2019099626A which describes a method for estimating a level of glucose concentration in the blood. The method includes the obtainment of a first and a second measurement of the glucose, the addition of the first and of the second glucose measurement to a set of data for the measurement of the glucose and the calculation of an estimated level of HbA1c. In addition, a method is provided for calculating an estimated HbA1 c level interval. The method comprises at least a calculation of an estimated HbA1c level and a standard deviation of the estimated HbA1c level by using a set of data for measuring the glucose and combining the estimated HbA1c level with the standard deviation of the estimated HbA1c level in order to acquire the HbA1c interval. In other words, the device for monitoring the glucose can monitor the glycemic variability of an individual.

Another example is the patent CA3077994A1 which describes a kit that provides methods and devices for measuring and managing the analyses in order to inform a diabetic patient regarding the glycemic conditions. The method provides for the execution of a measurement of the glucose before a meal with the analysis measurement and management device, in which the measurement of the glucose before the meal is marked in the memory of the analysis measurement and management device as the pre-meal glucose value. A measurement of the glucose is executed after a meal with the analysis measurement and management device, where the measurement of the glucose after the meal is marked in the memory of the device as post-meal value. The method also provides for determining if the difference between the post-meal glucose value and the pre-meal glucose value is lower than about 50 mg/dL and for warning the user each time the difference is greater than about 50 mg/dL. In addition, it reminds the user to repeat the test at a later time period.

None of the devices present today on the market allows the immediate access to reserves of glucose so as to not incur problems deriving from hypoglycemia crises. By not supplementing glucose within a proper time period, in fact, the risk of incurring irreversible consequences would remain unchanged. Therefore, due to the device described in detail hereinbelow, it will be possible to have an effective and immediate restoration of the glucose in any daily life situation so as to eliminate the risks tied to hypoglycemia, as well as a considerable psychological relief of the subjected affected with such deficiency.

Additional prior art can be found in patent literature US2010004522A1 and US10549037B2.

### Description of the invention

According to the present invention, the ergonomic, wearable smart device is attained, containing glucose and/or glucagon adapted to restore the concentration of glucose in the blood. Due to the capacity of this device to store predefined quantities of glucose and/or glucagon, due to its tubular shape or any other shape that renders it wearable and due to an advantageous magnetic closure, there is the possibility to quickly/suddenly respond to cases of hypoglycemia. Advantageously, in addition, situated on the surface of the device itself is a valve for reloading glucose, which occurs due to the advantageous use of a reload cartridge, in turn divided into: tank and lip (unit which will penetrate the reload valve, allowing the reloading itself).

Advantageously in order to facilitate the consumption of the glucose, there is a reversibly liftable straw, which at its interior houses a smart valve, which, advantageously being able to communicate via Bluetooth with latest-generation cellular device, CGM and insulin pumps, allows metering the liquid contained in the device and simultaneously prevents the leakage thereof. If the device housed predefined quantities of glucagon, however, it will be possible to consume it by means of a nasal spray integrated with the device. If instead the device was designed to house both glucose and glucagon, the device would be equipped both with straw and spray so as to make possible the consumption of both substances by the user.

In addition, the device is provided with a display with movement and/or touch interaction technology which provides a series of information benefiting the user, and can also interact with the aforesaid smart valve and with a glycemia detector, it too situated on the device, which can advantageously measure the levels of glucose in the blood and in turn communicate with the display.

In a further version, the device is capable of communicating with multiple smart-phones, it allows remote interactions and can signal important information to the user by means of sounds and vibrations as well as geolocate the user himself/herself and send SOS messages in case of need.

Furthermore and advantageously, an injection device is integratable, constituted by a retractable needle adapted to act as syringe, capable of being activated by the display or by multiple electronic devices, also remotely, due to the use of an application that was suitably designed for managing and controlling the entire device.

In a further advantageous version, the device can be composed of materials which render it impermeable, fireproof and underwater as well as having dark color so as to prevent the deterioration of the liquid at its interior and more generally allow the maintenance of a suitable temperature in order to prevent the crystallization or oxidation of the liquid itself.

Possibly, the device is made with recycled, recyclable and reusable materials designed such that they can fall within a circular economy.

### Description of the figures

The invention will be described hereinbelow in a preferred embodiment as a non-limiting example, with the aid of the enclosed figures, in which:

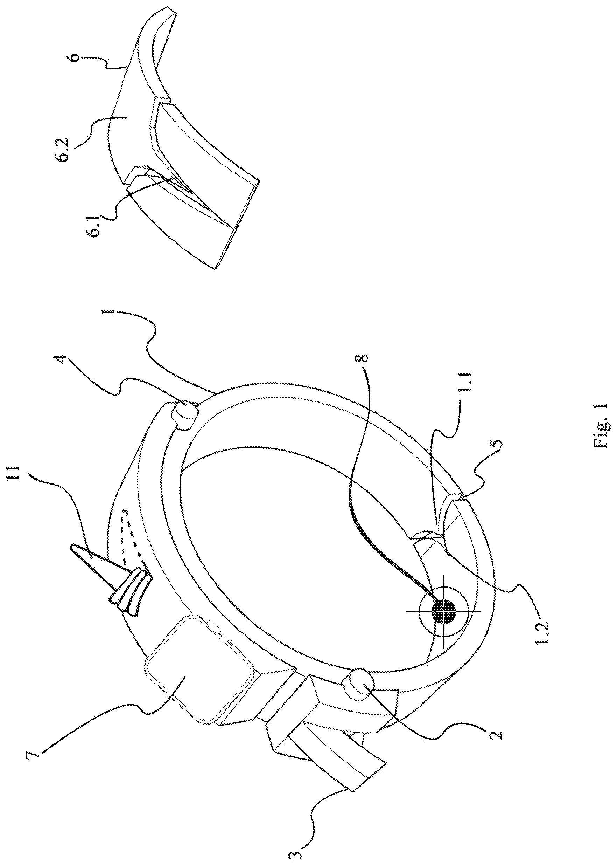

- FIGURE 1 shows several of the components of the present smart bracelet device: the body of the device 1 comprises a smart valve 2 inserted at the base of a straw 3 as well as a reload valve 4, a nasal spray 11, a closure 5 composed of a right end 1.1 and a left end 1.2, a reload cartridge 6 composed of a lip 6.1 and a tank 6.2; a display 7 and a glycemia detector 8 which are adjacent to each other.

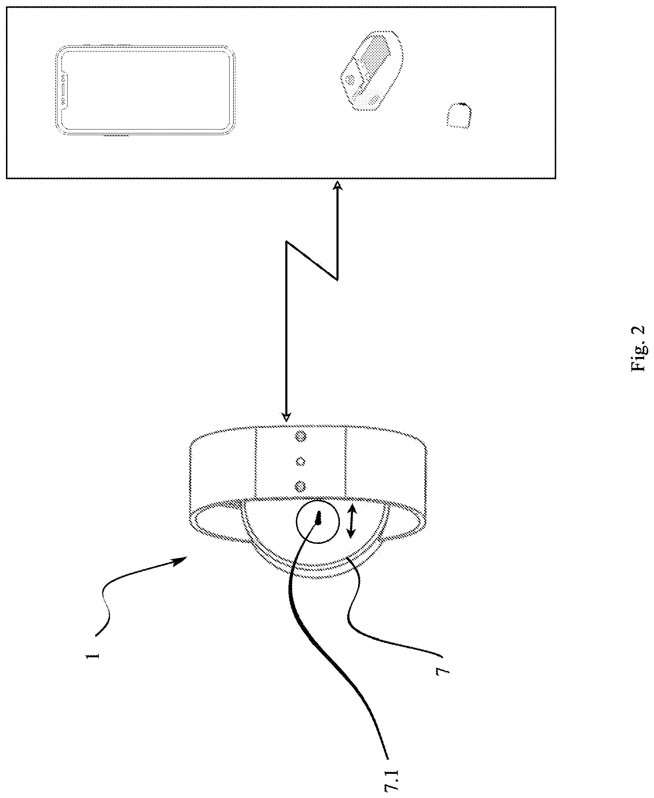

- FIGURE 2 schematically shows a bracelet device 1 according to the present invention in which in proximity to the display 7, the retractable needle 7.1. is placed, and the communication network between the display 7 and the connected portable devices is also shown.

### Detailed description of the invention

The present invention will now be illustrated merely as a non-limiting or non-constraining example, with reference to the figures which illustrate possible embodiments relative to the present inventive concept.

FIG. 1 shows the ergonomic, wearable smart device, containing glucose and/or glucagon in a preferred version: it is designed for restoring the concentration of glucose in the blood. The aforesaid wearable device is programmed for containing predetermined quantities of glucose and/or glucagon to be administered in case of episodes of severe hypoglycemia or hypoglycemia and comprises at least a body of the device 1 with tubular shape or with any other shape, adapted to contain predetermined quantities of glucose and/or glucagon. The aforesaid body of the device 1 is designed for being worn and fixed by means of a closure 5: this is designed for joining the two ends of the body of the device 1 which is designed for being reloaded by means of at least a reload valve 4 which allows the reloading of glucose and/or glucagon and simultaneously prevents the leakage thereof.

The aforesaid reload valve 4 is designed for allowing the insertion of at least a lip 6.1 of a reload cartridge 6; the valve 4, in addition, is designed for allowing the restoration of the reserves of glucose and/or glucagon, allowing the containment and the preservation of the same within the body device 1.

At least a straw 3, reversibly liftable, is placed above the aforesaid body of the device 1 and allows the consumption of a predetermined quantity of glucose.

In an embodiment variant, the device 1 of the present invention comprises glucagon and a retractable nasal spray 11 adapted to allow the nasal spraying of the content of the device 1, in case of need, by the user who wears the device on the wrist.

In a further variant, the aforesaid device 1 simultaneously comprises at its interior predefined quantities of glucose and glucagon, as well as at least a straw 3 and at least a nasal spray 11 which allow the consumption of both substances by the user.

In addition, at least a smart valve 2, placed inside the straw 3 and/or the nasal spray 11, which is able to be connected by means of Bluetooth to a latest-generation cellular device, CGM and insulin pump, receives information and allows the administration of the exact quantities to be administered as well as the preservation and containment of the glucose and/or glucagon contained within the aforesaid body of the device 1.

The present bracelet device is closed by means of the aforesaid magnetic closure 5 which is formed by two components: at least a metallic component placed on the right end 1.1 set on at least a magnetic component placed on the left end 1.2 and at least a magnetic component set on at least a metallic component placed on the right end 1.1.

The aforesaid bracelet device is also reloaded with glucose and/or glucagon by means of the aforesaid reload cartridge 6 comprising at least a tank 6.2 designed for containing predetermined quantities of glucose and/or glucagon and at least a lip 6.1 which allows the insertion of the aforesaid reload cartridge within the body of the device 1 through the aforesaid reload valve 4.

On the aforesaid body of the device 1, a display 7 is placed having preferably rectangular shape, which integrates a movement and/or touch interaction technology; such display 7 is designed for providing a plurality of data to the user, as it is also able to interact with the aforesaid smart valve 2, with latest-generation cellular device, CGM and insulin pumps. The display 7 can then communicate with a glycemia detector 8, also placed on said body of the device 1 and designed for checking the quantity of glucose in the blood and in turn communicating with the aforesaid display 7.

The present bracelet device is capable of communicating with multiple smartphones, allowing remote interactions and it integrates a geolocation device and SOS messaging in order to confront difficult moments as well as be able to produce sound alarms and vibrations.

As shown in Fig. 2, near the aforesaid display 7 an injection device 7.1 is placed that is constituted by a retractable needle adapted to act as syringe actuatable by means of the display 7 itself or even remotely through the use of a suitable developed application. In addition, the bracelet device is designed for being personalized upon request of any user, in a sport or luxury line or made of any other shape or color, in addition to the fact of being made with recycled materials and/or of natural origin designed for being able to fall within a circular economy. Alternatively, the present bracelet device is impermeable, fireproof and underwater, as well as rechargeable by means of solar energy. Finally, the bracelet device is thermoregulated and has dark color so as to prevent the deterioration of the liquid at its interior and to maintain a suitable and constant temperature so to avoid crystallizations and oxidations of the glucose.

## Claims

1. Ergonomic, smart wearable device, containing glucose and/or glucagon adapted to restore the concentration of glucose in the blood of a user; said wearable device being adapted to contain predetermined quantities of glucose to be administered in case of episodes of hypoglycemia to any one user; said wearable device comprising:
- at least a body of the device (1) with preferably tubular shape or in any case adapted to contain predetermined quantities of glucose; said body of the device (1) being adapted to be worn on the wrist of the user and fixed by means of a closure (5); said closure (5) being adapted to join the two ends of said body of the device (1); said body of the device (1) comprising at least a reload valve (4) which allows reinserting the predetermined quantity of glucose, preventing the accidental leakage thereof;
- at least said reload valve (4) adapted to house at least a lip (6.1) of a reload cartridge (6); said valve (4) being adapted to be traversed by at least a said reload cartridge (6) and being adapted for containing and preserving glucose within said body of the device (1);
- at least a straw (3), place above said body of the device (1), adapted for the user to consume a predetermined quantity of glucose; said straw (3) being reversibly liftable from said body of the device (1), allowing the oral administration of glucose for at least a user;
- at least a smart valve (2) placed inside said straw (3) being adapted to be connected by means of Bluetooth to a latest-generation cellular device, so as to receive information regarding the exact quantity to be administered to any one user and adapted for preserving and containing the glucose contained within said body of the device (1);
- at least a said magnetic closure (5), comprising two components, at least a metallic component placed on the right end (1.1) adapted to lie on at least a magnetic component placed on the left end (1.2) and at least a magnetic component adapted to lie on at least a metallic component placed on the right end (1.1); said closure (5) being adapted to close the two said ends (1.1, 1.2) of said body of the device (1);
- at least a said reload cartridge (6) comprising at least a tank (6.2) adapted to contain predetermined quantities of glucose and at least a lip (6.1) adapted to enter, by means of said reload valve (4), within said device body (1), allowing the restoration of glucose;
- at least a display (7) placed on said body of the device (1), integrating movement and/or touch interaction technology; said display (7) being adapted to provide a plurality of data and being adapted to interact with said smart valve (2);
- at least a glycemia detector (8) placed on said body of the device (1) adapted to check the quantity of glucose in the blood of the user adapted to interact with said display (7).

2. Ergonomic, smart wearable device, containing glucose and/or glucagon adapted to restore the concentration of glucose in the blood of a user; said wearable device being adapted to contain predetermined quantities of glucagon to be administered in case of episodes of severe hypoglycemia to any one user; said wearable device comprising :
- at least a body of the device (1) with preferably tubular form or in any case adapted to contain predetermined quantities of glucagon; said body of the device (1) being adapted to be worn on the wrist of the user and fixed by means of a closure (5); said closure (5) being adapted to join the two ends of said body of the device (1); said body of the device (1) comprising at least a reload valve (4) which allows restoring the predetermined quantity of glucagon, preventing the accidental leakage thereof;
- at least said reload valve (4) placed close to said closure (5) adapted to house at least a lip (6.1) of a reload cartridge (6); said valve (4) being adapted to be traversed by at least a said reload cartridge (6) and being adapted for containing and preserving glucagon within said body of the device (1);
- at least a retractable nasal spray (11), placed above said body of the device (1), adapted for the user to consume a predetermined quantity of glucagon; said nasal spray (11) being retractable by said body of the device (1), allowing the administration of glucagon through nose pathways to at least a user;
- at least a smart valve (2) placed inside said nasal spray (11), being adapted to be connected by means of Bluetooth to a latest-generation electronic device, so as to receive information regarding the exact quantity to be administered to any one user and adapted for preserving the glucagon contained within said body of the device (1);
- at least a said magnetic closure (5), comprising two components, at least a metallic component placed on the right end (1.1) adapted to lie on at least a magnetic component placed on the left end (1.2) and at least a magnetic component adapted to lie on at least a metallic component placed on the right end (1.1); said closure (5) being adapted to close the two said ends (1.1,1.2) of said body of the device (1);
- at least a said reload cartridge (6) comprising at least a tank (6.2) adapted to contain predetermined quantities of glucagon and at least a lip (6.1) adapted to enter by means of said reload valve (4) within said device body (1), allowing the restoration of glucagon;
- at least a display (7) placed on said body of the device (1), integrating movement and/or touch interaction technology; said display (7) being adapted to provide a plurality of data and being adapted to interact with said smart valve (2);
- at least a glycemia detector (8) placed on said body of the device (1) adapted to check the quantity of glucose in the blood of the user adapted to interact with said display (7).

3. Smart bracelet device containing glucose and glucagon, adapted to restore the concentration of glucose in the blood of a user; said wearable device being adapted to contain predetermined quantities of glucose and glucagon to be administered in case of episodes of hypoglycemia or severe hypoglycemia to any one user; said wearable device being **characterized in that** it comprises two separate internal tanks: one filled with glucose and a second filled with glucagon; said bracelet device being provided with the characteristics of claims 1 and 2, in particular comprising said nasal spray (11) at the tank of said glucagon and at least a said straw (3) at the tank of said glucose.

4. Smart bracelet device containing glucose and/or glucagon, according to any one of the preceding claims, **characterized in that** it comprises means for interconnection with one or more smartphones, CGM and insulin pumps and allowing remote interactions.

5. Smart bracelet device containing glucose and/or glucagon, according to the preceding claim, according to any one of the preceding claims, **characterized in that** it comprises a geolocation device and SOS messaging system for signaling in difficult conditions, as well as sound alarms and vibrations for signals directed to the user.

6. Smart bracelet device containing glucose and/or glucagon, according to the preceding claim, according to any one of the preceding claims, **characterized in that** it comprises at least an injection device (7.1), comprising a retractable needle, adapted to act as a syringe, manually controlled by means of said display (7) or automatically controlled by means of any one latest-generation electronic device, adapted to inject predetermined quantities of glucose and/or glucagon as a function of the detected data regarding glucose in the blood of the user.

7. Smart bracelet device containing glucose and/or glucagon, according to any one of the preceding claims, **characterized in that** it is personalized upon request of each user and is made in a sport or luxury line.

8. Smart bracelet device containing glucose and/or glucagon, according to any one of the preceding claims, **characterized in that** it is made with recycled materials and natural materials that can be reused once worn, in order to fall within a circular economy.

9. Smart bracelet device containing glucose and/or glucagon, according to any one of the preceding claims, **characterized in that** it comprises means for recharging via solar energy.

10. Smart bracelet device containing glucose and/or glucagon, according to any one of the preceding claims, **characterized in that** it comprises data connection means so as to interact with a dedicated application installable on latest-generation electronic devices.

11. Smart bracelet device containing glucose and/or glucagon, according to any one of the preceding claims, **characterized in that** it is impermeable, underwater and fireproof so as to allow the use thereof in every condition, and it also has dark color in order to prevent the deterioration of the glucose and/or glucagon due to solar rays.

12. Smart bracelet device containing glucose and/or glucagon, according to any one of the preceding claims, **characterized in that** it comprises thermoregulation means so as to maintain the temperature of the glucose and/or glucagon within pre-established parameters such to prevent the crystallization and oxidation of the glucose.

## Patentansprüche

1. Ergonomische, intelligente, tragbare Vorrichtung, die Glukose und/oder Glukagon enthält, die angepasst ist, um die Glukosekonzentration im Blut eines Benutzers wiederherzustellen; wobei die tragbare Vorrichtung angepasst ist, um vorbestimmte Mengen an Glukose zu enthalten, die im Falle von Hypoglykämie-Episoden an einen beliebigen Benutzer zu verabreichen sind; die tragbare Vorrichtung umfassend:
- mindestens einen Körper der Vorrichtung (1) mit vorzugsweise rohrförmiger Form oder der in jedem Fall angepasst ist, um vorbestimmte Mengen von Glukose zu enthalten; wobei der Körper der Vorrichtung (1) angepasst ist, um am Handgelenk des Benutzers getragen zu werden und mittels eines Verschlusses (5) befestigt zu werden; der Verschluss (5) angepasst ist, um die zwei Enden des Körpers der Vorrichtung (1) zu verbinden; der Körper der Vorrichtung (1) umfassend mindestens ein Nachladeventil (4), das unter Verhinderung eines unbeabsichtigten Auslaufens davon ein Wiedereinführen der vorbestimmten Menge an Glukose ermöglicht;
- mindestens das Nachladeventil (4) angepasst ist, um mindestens eine Lippe (6.1) einer Nachladekartusche (6) aufzunehmen; wobei das Ventil (4) angepasst ist, um von mindestens einer von der Nachladekartusche (6) durchquert zu werden und angepasst ist, um Glukose im Körper der Vorrichtung (1) zu enthalten und zu bewahren;
- mindestens einen Strohhalm (3), der oberhalb des Körpers der Vorrichtung (1) platziert ist, der angepasst ist, damit der Benutzer eine vorbestimmte Menge an Glukose konsumiert; wobei der Strohhalm (3) reversibel von dem Körper der Vorrichtung (1) abhebbar ist, was die orale Verabreichung von Glukose für mindestens einen Benutzer ermöglicht;
- mindestens ein intelligentes Ventil (2), das im Inneren des Strohhalms (3) platziert und angepasst ist, um mittels Bluetooth mit einer Mobilfunkvorrichtung der neuesten Generation verbunden zu werden, um Informationen in Bezug auf die genaue Menge zu erhalten, die einem beliebigen Benutzer verabreicht werden soll, und angepasst ist, um die im Körper der Vorrichtung (1) enthaltene Glukose zu bewahren und zu enthalten;
- mindestens einen magnetischen Verschluss (5), umfassend zwei Komponenten, wovon mindestens eine metallische Komponente, die an dem rechten Ende (1.1) platziert ist, angepasst ist, um auf mindestens einer magnetischen Komponente an dem linken Ende (1.2) zu liegen, und mindestens eine magnetische Komponente, die angepasst ist, um auf mindestens einer metallischen Komponente zu liegen, die an dem rechten Ende (1.1) platziert ist; wobei der Verschluss (5) angepasst ist, um die beiden Enden (1.1, 1.2) des Körpers der Vorrichtung (1) zu schließen;
- mindestens eine der Nachladekartuschen (6), umfassend mindestens einen Tank (6.2), der angepasst ist, um vorbestimmte Mengen an Glukose zu enthalten, und mindestens eine Lippe (6.1), die angepasst ist, um mittels des Nachladeventils (4) in den Vorrichtungskörper (1) einzutreten, was die Wiederherstellung von Glukose ermöglicht;
- mindestens eine Anzeige (7), die auf dem Körper der Vorrichtung (1) platziert ist, die eine Bewegungs- und/oder Berührungsinteraktionstechnologie integriert; wobei die Anzeige (7) angepasst ist, um eine Vielzahl von Daten bereitzustellen, und angepasst ist, um mit dem intelligenten Ventil (2) zu interagieren;
- mindestens einen Glykämie-Detektor (8), der auf dem Körper der Vorrichtung (1) platziert und angepasst ist, um die Menge an Glukose im Blut des Benutzers zu überprüfen, und der angepasst ist, um mit der Anzeige (7) zu interagieren.

2. Ergonomische, intelligente, tragbare Vorrichtung, die Glukose und/oder Glukagon enthält, die angepasst ist, um die Glukosekonzentration im Blut eines Benutzers wiederherzustellen; wobei die tragbare Vorrichtung angepasst ist, um vorbestimmte Mengen an Glukagon zu enthalten, die im Falle von Episoden schwerer Hypoglykämie an einen beliebigen Benutzer zu verabreichen sind; die tragbare Vorrichtung umfassend:
∘ mindestens einen Körper der Vorrichtung (1) mit vorzugsweise rohrförmiger Form oder der in jedem Fall angepasst ist, um vorbestimmte Mengen von Glukagon zu enthalten; wobei der Körper der Vorrichtung (1) angepasst ist, um am Handgelenk des Benutzers getragen zu werden und mittels eines Verschlusses (5) befestigt zu werden; der Verschluss (5) angepasst ist, um die zwei Enden des Körpers der Vorrichtung (1) zu verbinden; der Körper der Vorrichtung (1) umfassend mindestens ein Nachladeventil (4), das unter Verhinderung eines unbeabsichtigten Auslaufens davon ein Wiederherstellen der vorbestimmten Menge an Glukagon ermöglicht;
- mindestens das Nachladeventil (4) in der Nähe des Verschlusses (5) platziert ist und angepasst ist, um mindestens eine Lippe (6.1) einer Nachladekartusche (6) aufzunehmen; wobei das Ventil (4) angepasst ist, um von mindestens einer von der Nachladekartusche (6) durchquert zu werden und angepasst ist, um Glukagon im Körper der Vorrichtung (1) zu enthalten und zu bewahren;
- mindestens ein einziehbares Nasenspray (11), das oberhalb des Körpers der Vorrichtung (1) platziert und angepasst ist, damit der Benutzer eine vorbestimmte Menge an Glukagon konsumiert; wobei das Nasenspray (11) durch den Körper der Vorrichtung (1) einziehbar ist, was die Verabreichung von Glukagon durch die Nasenwege an mindestens einen Benutzer ermöglicht;
- mindestens ein intelligentes Ventil (2), das im Inneren des Nasensprays (11) platziert und angepasst ist, um mittels Bluetooth mit einer elektronischen Vorrichtung der neuesten Generation verbunden zu werden, um Informationen in Bezug auf die genaue Menge zu erhalten, die einem beliebigen Benutzer verabreicht werden soll, und angepasst ist, um das im Körper der Vorrichtung (1) enthaltene Glukagon zu bewahren;
- mindestens einen magnetischen Verschluss (5), umfassend zwei Komponenten, wovon mindestens eine metallische Komponente, die an dem rechten Ende (1.1) platziert ist, angepasst ist, um auf mindestens einer magnetischen Komponente an dem linken Ende (1.2) zu liegen, und mindestens eine magnetische Komponente, die angepasst ist, um auf mindestens einer metallischen Komponente zu liegen, die an dem rechten Ende (1.1) platziert ist; wobei der Verschluss (5) angepasst ist, um die beiden Enden (1.1, 1.2) des Körpers der Vorrichtung (1) zu schließen;
- mindestens eine der Nachladekartuschen (6), umfassend mindestens einen Tank (6.2), der angepasst ist, um vorbestimmte Mengen an Glukagon zu enthalten, und mindestens eine Lippe (6.1), die angepasst ist, um mittels des Nachladeventils (4) in den Vorrichtungskörper (1) einzutreten, was die Wiederherstellung von Glukagon ermöglicht;
- mindestens eine Anzeige (7), die auf dem Körper der Vorrichtung (1) platziert ist, die eine Bewegungs- und/oder Berührungsinteraktionstechnologie integriert; wobei die Anzeige (7) angepasst ist, um eine Vielzahl von Daten bereitzustellen, und angepasst ist, um mit dem intelligenten Ventil (2) zu interagieren;
- mindestens einen Glykämie-Detektor (8), der auf dem Körper der Vorrichtung (1) platziert und angepasst ist, um die Menge an Glukose im Blut des Benutzers zu überprüfen, und der angepasst ist, um mit der Anzeige (7) zu interagieren.

3. Intelligentes Armband, das Glukose und Glukagon enthält, das angepasst ist, um die Glukosekonzentration im Blut eines Benutzers wiederherzustellen; wobei die tragbare Vorrichtung angepasst ist, um vorbestimmte Mengen an Glukose und Glukagon zu enthalten, die im Falle von Episoden von Hypoglykämie oder schwerer Hypoglykämie an einen beliebigen Benutzer zu verabreichen sind; wobei die tragbare Vorrichtung **dadurch gekennzeichnet ist, dass** sie zwei separate interne Behälter umfasst: einen gefüllt mit Glukose und einen zweiten gefüllt mit Glukagon; wobei die Armbandvorrichtung mit den Merkmalen der Ansprüche 1 und 2 versehen ist, insbesondere umfassend das Nasenspray (11) an dem Behälter des Glukagons und mindestens den Strohhalm (3) an dem Behälter der Glukose.

4. Intelligente Armbandvorrichtung, die Glukose und/oder Glukagon enthält, nach einem der vorherigen Ansprüche, **dadurch gekennzeichnet, dass** sie Einrichtungen zur Verbindung mit einem oder mehreren Smartphones, CGM und Insulinpumpen umfasst und ferngesteuerte Interaktionen ermöglicht.

5. Intelligente Armbandvorrichtung, die Glukose und/oder Glukagon enthält, nach dem vorherigen Anspruch, nach einem der vorherigen Ansprüche, **dadurch gekennzeichnet, dass** sie eine Geolokalisierungsvorrichtung und ein SOS-Meldesystem zum Signalisieren unter schwierigen Bedingungen sowie akustische Alarme und Vibrationen für an den Benutzer gerichtete Signale umfasst.

6. Intelligente Armbandvorrichtung, die Glukose und/oder Glukagon enthält, nach dem vorherigen Anspruch, nach einem der vorherigen Ansprüche, **dadurch gekennzeichnet, dass** sie mindestens eine Injektionsvorrichtung (7.1) umfasst, umfassend eine einziehbare Nadel, die angepasst ist, um als Spritze zu fungieren, die manuell über die Anzeige (7) oder automatisch über eine beliebige elektronische Vorrichtung der neuesten Generation gesteuert wird und die angepasst ist, um vorbestimmte Mengen an Glukose und/oder Glukagon abhängig von den erfassten Daten in Bezug auf Glukose im Blut des Benutzers zu injizieren.

7. Intelligente Armbandvorrichtung, die Glukose und/oder Glukagon enthält, nach einem der vorherigen Ansprüche, **dadurch gekennzeichnet, dass** sie auf Wunsch eines jeden Benutzers personalisiert und in einer Sport- oder Luxuslinie gefertigt wird.

8. Intelligente Armbandvorrichtung, die Glukose und/oder Glukagon enthält, nach einem der vorherigen Ansprüche, **dadurch gekennzeichnet, dass** sie aus recycelten Materialien und natürlichen Stoffen gefertigt wird, die nach dem Tragen wiederverwendet werden können, um der Kreislaufwirtschaft zugeführt zu werden.

9. Intelligente Armbandvorrichtung, die Glukose und/oder Glukagon enthält, nach einem der vorherigen Ansprüche, **dadurch gekennzeichnet, dass** sie Einrichtungen zum Aufladen durch Sonnenenergie umfasst.

10. Intelligente Armbandvorrichtung, die Glukose und/oder Glukagon enthält, nach einem der vorherigen Ansprüche, **dadurch gekennzeichnet, dass** sie Datenverbindungseinrichtungen umfasst, um mit einer dedizierten Anwendung zu interagieren, die auf elektronischen Vorrichtungen der neuesten Generation installierbar ist.

11. Intelligente Armbandvorrichtung, die Glukose und/oder Glukagon enthält, nach einem der vorherigen Ansprüche, **dadurch gekennzeichnet, dass** sie unter Wasser undurchlässig und feuerfest ist, um deren Verwendung unter allen Bedingungen zu ermöglichen, und dass sie auch eine dunkle Farbe aufweist, um die Zustandsverschlechterung der Glukose und/oder des Glukagons aufgrund von Sonnenstrahlen zu verhindern.

12. Intelligente Armbandvorrichtung, die Glukose und/oder Glukagon enthält, nach einem der vorherigen Ansprüche, **dadurch gekennzeichnet, dass** sie Wärmeregulierungseinrichtungen umfasst, um die Temperatur der Glukose und/oder des Glukagons innerhalb vorher festgelegter Parameter zu halten, um die Kristallisation und Oxidation der Glukose zu verhindern.

## Revendications

1. Dispositif portable ergonomique et intelligent, contenant du glucose et/ou du glucagon, adapté pour rétablir la concentration en glucose dans le sang d'un utilisateur, ledit dispositif portable étant adapté pour contenir des quantités prédéterminées de glucose à administrer en cas d'épisodes d'hypoglycémie à n'importe quel utilisateur ; ledit dispositif portable comprenant :
- au moins un corps du dispositif (1) de préférence en forme tubulaire ou en tout cas adapté pour contenir des quantités prédéterminées de glucose, ledit corps du dispositif (1) étant adapté pour être porté au poignet de l'utilisateur et fixé au moyen d'une fermeture (5) ; ladite fermeture (5) étant adaptée pour relier les deux extrémités dudit corps du dispositif (1) ; ledit corps du dispositif (1) comprenant au moins une vanne de recharge (4) qui permet de réinsérer la quantité prédéterminée de glucose, en empêchant toute fuite accidentelle de celle-ci ;
- au moins ladite vanne de recharge (4) étant adaptée pour loger au moins une lèvre (6.1) d'une cartouche de recharge (6) ; ladite vanne (4) étant adaptée pour être traversée au moins ladite cartouche de rechargement (6) et étant adaptée pour contenir et conserver le glucose à l'intérieur dudit corps du dispositif (1) ;
- au moins une paille (3), placée au-dessus dudit corps du dispositif (1), adaptée pour permettre à l'utilisateur de consommer une quantité prédéterminée de glucose ; ladite paille (3) pouvant être retirée de manière réversible dudit corps du dispositif (1), permettant ainsi d'administrer oralement le glucose à au moins un utilisateur,
- au moins une vanne intelligente (2) placée à l'intérieur de ladite paille (3) étant adaptée pour être connectée par Bluetooth à un appareil cellulaire de dernière génération, de manière à recevoir des informations concernant la quantité exacte à administrer à un utilisateur donné et adaptée pour conserver et contenir le glucose contenu dans ledit corps de l'appareil (1),
- au moins ladite fermeture magnétique (5), comprenant deux composants, au moins un composant métallique placé sur l'extrémité droite (1.1) adapté pour reposer sur au moins un composant magnétique placé sur l'extrémité gauche (1.2) et au moins un composant magnétique adapté pour reposer sur au moins un composant métallique placé sur l'extrémité droite (1.1) ; ladite fermeture (5) étant adaptée pour fermer lesdites deux extrémités (1.1, 1.2) dudit corps du dispositif (1) ;
- au moins ladite cartouche de recharge (6) comprenant au moins un réservoir (6.2) adapté pour contenir des quantités prédéterminées de glucose et au moins une lèvre (6.1) adaptée pour entrer, au moyen de ladite vanne de recharge (4), à l'intérieur dudit corps de dispositif (1), permettant la restauration du glucose ;
- au moins un écran (7) placé sur ledit corps du dispositif (1), intégrant une technologie de mouvement et/ou d'interaction tactile ; ledit écran (7) étant adapté pour fournir une pluralité de données et étant adapté pour interagir avec ladite vanne intelligente (2),
- au moins un détecteur de glycémie (8) placé sur ledit corps du dispositif (1), adapté pour vérifier la quantité de glucose dans le sang de l'utilisateur, adapté pour interagir avec ledit écran (7).

2. Dispositif portable ergonomique et intelligent, contenant du glucose et/ou du glucagon, adapté pour rétablir la concentration en glucose dans le sang d'un utilisateur, ledit dispositif portable étant adapté pour contenir des quantités prédéterminées de glucagon à administrer en cas d'épisodes d'hypoglycémie grave à n'importe quel utilisateur ; ledit dispositif portable comprenant :
- au moins un corps du dispositif (1) de préférence en forme tubulaire ou en tout cas adapté pour contenir des quantités prédéterminées de glucagon, ledit corps du dispositif (1) étant adapté pour être porté au poignet de l'utilisateur et fixé au moyen d'une fermeture (5) ; ladite fermeture (5) étant adaptée pour relier les deux extrémités dudit corps du dispositif (1) ; ledit corps du dispositif (1) comprenant au moins une vanne de recharge (4) qui permet de restaurer la quantité prédéterminée de glucagon, en empêchant toute fuite accidentelle de celle-ci ;
- au moins ladite vanne de recharge (4) placée près de ladite fermeture (5) adaptée pour loger au moins une lèvre (6.1) d'une cartouche de recharge (6) ; ladite vanne (4) étant adaptée pour être traversée au moins ladite cartouche de rechargement (6) et étant adaptée pour contenir et conserver le glucagon à l'intérieur dudit corps du dispositif (1) ;
- au moins un spray nasal rétractable (11), placé au-dessus dudit corps du dispositif (1), adapté pour permettre à l'utilisateur de consommer une quantité prédéterminée de glucagon ; ledit un spray nasal (11) étant rétractable par ledit corps du dispositif (1), permettant ainsi d'administrer le glucagon par les voies nasales à au moins un utilisateur ;
- au moins une vanne intelligente (2) placée à l'intérieur dudit spray nasal (11) étant adaptée pour être connectée par Bluetooth à un appareil électronique de dernière génération, de manière à recevoir des informations concernant la quantité exacte à administrer à un utilisateur donné et adaptée pour conserver le glucagon contenu dans ledit corps de l'appareil (1),
- au moins ladite fermeture magnétique (5), comprenant deux composants, au moins un composant métallique placé sur l'extrémité droite (1.1) adapté pour reposer sur au moins un composant magnétique placé sur l'extrémité gauche (1.2) et au moins un composant magnétique adapté pour reposer sur au moins un composant métallique placé sur l'extrémité droite (1.1) ; ladite fermeture (5) étant adaptée pour fermer lesdites deux extrémités (1.1, 1.2) dudit corps du dispositif (1) ;
- au moins ladite cartouche de recharge (6) comprenant au moins un réservoir (6.2) adapté pour contenir des quantités prédéterminées de glucagon et au moins une lèvre (6.1) adaptée pour entrer, au moyen de ladite vanne de recharge (4), à l'intérieur dudit corps de dispositif (1), permettant la restauration du glucagon ;
- au moins un écran (7) placé sur ledit corps du dispositif (1), intégrant une technologie de mouvement et/ou d'interaction tactile ; ledit écran (7) étant adapté pour fournir une pluralité de données et étant adapté pour interagir avec ladite vanne intelligente (2) ;
- au moins un détecteur de glycémie (8) placé sur ledit corps du dispositif (1), adapté pour vérifier la quantité de glucose dans le sang de l'utilisateur, adapté pour interagir avec ledit écran (7).

3. Bracelet intelligent contenant du glucose et du glucagon, adapté pour rétablir la concentration de glucose dans le sang d'un utilisateur ; ledit dispositif portable étant adapté pour contenir des quantités prédéterminées de glucose et de glucagon à administrer en cas d'épisodes d'hypoglycémie ou d'hypoglycémie sévère à n'importe quel utilisateur, ledit dispositif portable étant **caractérisé en ce qu'**il comprend deux réservoirs internes séparés : l'un rempli de glucose et le deuxième rempli de glucagon ; ledit bracelet présentant les caractéristiques selon les revendications 1 et 2, comprenant en particulier ledit spray nasal (11) au niveau du réservoir dudit glucagon et au moins ladite paille (3) au niveau du réservoir dudit glucose.

4. Bracelet intelligent contenant du glucose et/ou du glucagon, selon l'une quelconque des revendications précédentes, **caractérisé en ce qu'**il comprend des moyens d'interconnexion avec un ou plusieurs smartphones, CGM et pompes à insuline et permettant des interactions à distance.

5. Bracelet intelligent contenant du glucose et/ou du glucagon, selon la revendication précédente, selon l'une quelconque des revendications précédentes, **caractérisé en ce qu'**il comprend un dispositif de géolocalisation et un système de messagerie SOS permettant d'envoyer des signaux dans des conditions difficiles, ainsi que des alarmes sonores et des vibrations pour les signaux destinés à l'utilisateur.

6. Bracelet intelligent contenant du glucose et/ou du glucagon, selon la revendication précédente, selon l'une quelconque des revendications précédentes, **caractérisé en ce qu'**il comprend au moins un dispositif d'injection (7.1), comprenant une aiguille rétractable, adapté pour agir comme une seringue, commandé manuellement au moyen dudit écran (7) ou commandé automatiquement au moyen d'un dispositif électronique de dernière génération, adapté pour injecter des quantités prédéterminées de glucose et/ou de glucagon en fonction des données détectées concernant le glucose dans le sang de l'utilisateur.

7. Bracelet intelligent contenant du glucose et/ou du glucagon, selon l'une quelconque des revendications précédentes, **caractérisé en ce qu'**il est personnalisé à la demande de chaque utilisateur et est fabriqué dans une gamme sport ou luxe.

8. Bracelet intelligent contenant du glucose et/ou du glucagon, selon l'une quelconque des revendications précédentes, **caractérisé en ce qu'**il est fabriqué à partir de matériaux recyclés et de matériaux naturels pouvant être réutilisés une fois usagés, afin de s'inscrire dans une économie circulaire.

9. Bracelet intelligent contenant du glucose et/ou du glucagon, selon l'une quelconque des revendications précédentes, **caractérisé en ce qu'**il comprend des moyens de recharge par l'énergie solaire.

10. Bracelet intelligent contenant du glucose et/ou du glucagon, selon l'une quelconque des revendications précédentes, **caractérisé en ce qu'**il comprend des moyens de connexion de données afin d'interagir avec une application dédiée pouvant être installée sur des appareils électroniques de dernière génération.

11. Bracelet intelligent contenant du glucose et/ou du glucagon, selon l'une quelconque des revendications précédentes, **caractérisé en ce qu'**il est imperméable, submersible et ignifuge afin de pouvoir être utilisé dans toutes les conditions, et sa couleur sombre permet également d'empêcher la détérioration du glucose et/ou du glucagon du fait des rayons solaires.

12. Bracelet intelligent contenant du glucose et/ou du glucagon, selon l'une quelconque des revendications précédentes, **caractérisé en ce qu'**il comprend des moyens de thermorégulation permettant de maintenir la température du glucose et/ou du glucagon dans des paramètres préétablis afin d'empêcher la cristallisation et l'oxydation du glucose.
